(19) Europäisches Patentamt / European Patent Office / Office européen des brevets

(11) **EP 4 470 463 A1**

(12) **EUROPEAN PATENT APPLICATION**

(43) Date of publication:
**04.12.2024 Bulletin 2024/49**

(21) Application number: **24178768.8**

(22) Date of filing: **29.05.2024**

(51) International Patent Classification (IPC):
**A61B 5/1455** *(2006.01)*    **A61B 5/024** *(2006.01)*
**A61B 5/18** *(2006.01)*    **A61B 5/00** *(2006.01)*

(52) Cooperative Patent Classification (CPC):
**A61B 5/18; A61B 5/1455; A61B 5/14551;**
**A61B 5/6893;** A61B 5/02416; A61B 5/02433;
A61B 5/7221; A61B 2503/22; A61B 2562/125

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **31.05.2023 KR 20230070470**

(71) Applicant: **LG Electronics Inc.**
**Yeongdeungpo-gu**
**Seoul 07336 (KR)**

(72) Inventor: **RHEE, Byungjoon**
**06772 Seoul (KR)**

(74) Representative: **Vossius & Partner**
**Patentanwälte Rechtsanwälte mbB**
**Siebertstrasse 3**
**81675 München (DE)**

(54) **DRIVER MONITORING DEVICE AND METHOD FOR OPERATING DRIVER MONITORING DEVICE**

(57)  A driver monitoring device and a method for operating the driver monitoring device are disclosed. A method for controlling a driver monitoring system according to at least one of various embodiments of the present disclosure may include detecting a user; obtaining sensing data for the detected user; converting the sensing data when a difference between the sensing data and predetermined reference data is equal to or greater than a threshold value; and generating and transmitting a vehicle control signal based on the converted sensing data.

## FIG. 1

Description

**The Background**

**1. The field**

The present disclosure relates to a driver monitoring device and a method for operating the driver monitoring device.

**2. Description of the related art**

A Vehicle driver monitoring system (DMS) is a system designed to enhance driver safety and it is a technology that monitors the driver's condition by detecting the driver's face, eye movement, head movement, and hand movement.
This vehicle DMS detects the driver's drowsy driving, reduced attention, drunk driving, or the like is detected and contributes to accident prevention by recognizing them in advance. In addition, the vehicle DMS can detect whether the driver is wearing a seatbelt and take actions such as sounding a warning sound or automatically stopping the vehicle when abnormal signs are detected.
A conventional vehicle DMS uses a camera recognition method or a biometric recognition method.
However, in the case of the camera recognition method, it is difficult to accurately determine when the driver moves a lot, and the accuracy is low because it is a technology that analyzes pulse waves through displacement values returned after IR is reflected on the driver.
In addition, in the case of the biometric recognition method, the pulse wave analysis has a large influence on the driver's driving habit, so it is difficult to establish, and there is an issue that the electrocardiogram must be implemented in a non-contact method, which has become a problem.

**The Summary**

An object of the present disclosure is to provide a driver monitoring device and method that allows a driver monitoring system (DMS) to be implemented immediately when a driver gets into a vehicle and sits on a seat.
An object of the present disclosure is to provide a driver monitoring device and method for guaranteeing accuracy while minimizing a driver's burden when applying a DMS to a vehicle.
A method for controlling a driver monitoring system according to at least one of various embodiments of the present disclosure includes detecting a user; obtaining sensing data for the detected user; converting the sensing data when a difference between the sensing data and predetermined reference data is equal to or greater than a threshold value; and generating and transmitting a first vehicle control signal based on the converted sensing data, wherein the sensing data may be obtained through at least one contact sensor provided in the vehicle.
According to the method for controlling a driver monitoring system according to the present disclosure, the at least one contact sensor may be formed using a fabric electronic yarn.
According to the method for controlling a driver monitoring system according to the present disclosure, the at least one contact sensor may be built on a handle of the vehicle.
According to the method for controlling a driver monitoring system according to the present disclosure, the at least one contact sensor may be disposed on the handle of the vehicle at predetermined intervals and may be disposed in an area other than the extended portion of a frame of the handle.
According to the method for controlling a driver monitoring system according to the present disclosure, the contact sensor may include of a plurality of sensors, light receivers, and signal generators.
According to the method for controlling a driver monitoring system according to the present disclosure, the plurality of sensors may include one Mid-Infrared (MIR) sensor, one Near-Infrared (NIR) sensor, and a sensor generating a first wavelength.
According to the method for controlling a driver monitoring system according to the present disclosure, the plurality of sensors may include one MIR sensor, one NIR sensor, a sensor generating a first wavelength, and a sensor generating a second wavelength.
According to the method for controlling a driver monitoring system according to the present disclosure, the plurality of sensors may include one IR sensor, a sensor generating a first wavelength and a sensor generating a second wavelength.
According to the method for controlling a driver monitoring system according to the present disclosure, the contact sensor disposed on the handle may have different configurations and placement intervals of each sensor according to a placement area.
According to the method for controlling a driver monitoring system according to the present disclosure, the plurality of sensors may include at least one of a first sensor of a first wavelength for measuring oxygen saturation, a second sensor of a second wavelength for measuring ethanol concentration, and a third sensor of a third wavelength for measuring pulse wave.

The method for controlling a driver monitoring system according to the present disclosure may further include monitoring whether an access signal is sensed in the vehicle.

The method for controlling a driver monitoring system according to the present disclosure may further include, as a result of the monitoring, when an access signal is sensed in the vehicle, and sensing data is not obtained for a predefined time from at least one contact sensor disposed on the handle of the vehicle at predetermined intervals, generating and transmitting a second vehicle control signal.

According to the method for controlling a driver monitoring system according to the present disclosure, the at least one contact sensor may be built in at least one of a start button of the vehicle, a button built in the vehicle, or a button on the vehicle remote control device.

According to the method for controlling a driver monitoring system according to the present disclosure, the at least one contact sensor may include at least one of a first sensor of a first wavelength for measuring oxygen saturation, a second sensor of a second wavelength for measuring ethanol concentration, or a third sensor of a third wavelength for measuring pulse wave.

According to an apparatus for controlling a driver monitoring system according to the present disclosure, the apparatus may include a sensor portion configured to detect a user and obtains sensing data for the detected user; and a processor configured to, when the difference between the sensing data and predetermined reference data is equal to or greater than a threshold value, convert the sensing data and generate and transmit a vehicle control signal based on the converted sensing data, wherein the sensing data may be obtained through at least one contact sensor provided in the vehicle.

According to at least one of various embodiments of the present disclosure, there is an effect that a driver monitoring device and method can be provided so that a driver monitoring system (DMS) is performed immediately when a driver gets into a vehicle and sits on a seat.

According to at least one of various embodiments of the present disclosure, there is an effect that a driver monitoring device and method can be provided which guarantees accuracy while minimizing a driver's burden.

## BRIEF DESCRIPTION OF THE DRAWINGS

FIG. 1 is a schematic diagram illustrating a driver monitoring system according to an embodiment of the present disclosure.

FIG. 2 is a configuration block diagram illustrating the driver monitoring device of FIG. 1.

FIG. 3 is a flowchart for explaining a driver monitoring method according to an embodiment of the present disclosure.

FIG. 4 is a diagram for explaining application of a non-contact monitoring technology according to an embodiment of the present disclosure to clothes and a smart band.

FIGS. 5 and 6 are diagrams for explaining application of a non-contact monitoring technology according to an embodiment of the present disclosure to a vehicle.

FIG. 7 illustrates a smart pad built into a vehicle seat and clothes according to an embodiment of the present disclosure.

FIGS. 8 to 11 explain the structure of an electronic yarn forming a smart pad according to an embodiment of the present disclosure.

FIG. 12 is a diagram for explaining the formation of a smart pad and a driving portion of an ECG, BTM, and PPG monitoring system according to an embodiment of the present disclosure.

FIG. 13 is a diagram for explaining a method of directly mounting a driving board on fabric according to an embodiment of the present disclosure.

FIG. 14 is a diagram for explaining the formation of the electronic yarn through a method of forming a yarn through a spinning process after manufacturing a core wiring substrate according to an embodiment of the present disclosure.

FIG. 15 is a diagram for explaining a method of manufacturing an electronic yarn used as a smart pad according to an embodiment of the present disclosure.

FIGS. 16 and 17 are diagrams for explaining an E-field collector for building a non-contact type monitoring system according to an embodiment of the present disclosure.

FIG. 18 explains a state of wearing the PPG sensor according to an embodiment of the present disclosure and the principle thereof.

FIG. 19 is a diagram for explaining ethanol reflectance spectrum characteristics in a DMS using a fabric electronic yarn-based PPG sensor according to an embodiment of the present disclosure.

FIG. 20 is a diagram for explaining the MIR transmission spectrum illustrated in FIG. 19(a).

FIG. 21 is a diagram for explaining the NIR transmission spectrum illustrated in FIG. 19(b).

FIG. 22 is a diagram for explaining an example in which an electronic yarn-based PPG sensor is mounted on a handle of a vehicle according to an embodiment of the present disclosure.

FIG. 23 is a diagram for explaining a wire bonding condition in which each sensor discontinuously disposed on a handle according to an embodiment of the present disclosure may be wire bonded.

FIG. 24 is a configuration block diagram illustrating a driver monitoring system including a PPG sensor formed based on the electronic yarn according to an embodiment of the present disclosure.

FIG. 25 is a view for explaining an embodiment referring to a button provided in a vehicle rather than a handle of FIG. 18 and below described above.

## The Detailed description

Hereinafter, embodiments related to the present disclosure will be described in more detail with reference to the drawings. The suffixes "module" and "portion" for components used in the following description are given or used together in consideration of ease of writing the specification, and do not have meanings or roles that are distinct from each other by themselves.

A monitoring device (or system) according to various embodiment(s) of the present disclosure may be built into a vehicle, for example. In this case, the monitoring device may be named and described as a driver monitoring device.

Meanwhile, the driver monitoring device according to the present disclosure may be a driver monitoring system (DMS) or a portion thereof. However, the present disclosure is not limited thereto. For example, the driver monitoring device may be a separate component for DMS or a device in a comprehensive sense including DMS.

Hereinafter, a smart pad (for example, ECG pad)-based driver monitoring device (or system) using an electronic yarn, a sensor pad using the electronic yarn, and a vehicle including the sensor pad will be described.

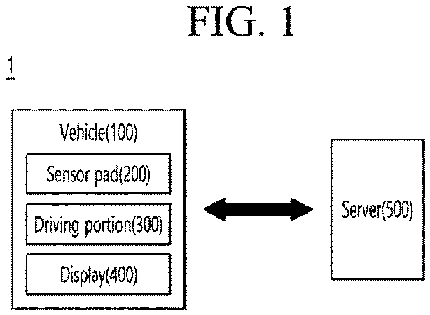

FIG. 1 is a schematic diagram illustrating a driver monitoring system 1 according to an embodiment of the present disclosure.

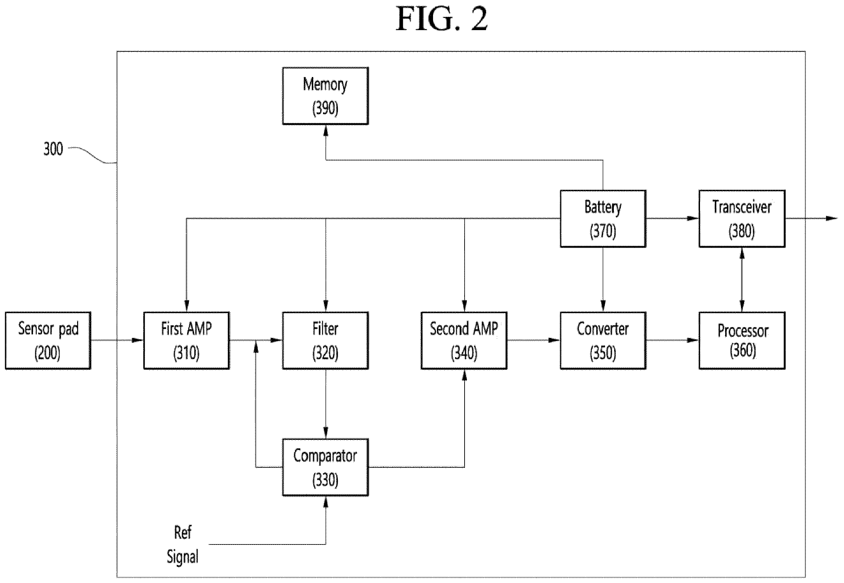

FIG. 2 is a configuration block diagram illustrating the driver monitoring device of FIG. 1.

Referring to FIG. 1, a driver monitoring system 1 may include a vehicle 100 and a server 500.

The server 500 may not be an essential component. The server 500 may receive or collect data of the vehicle 100 and process it.

In particular, when data is received from the vehicle 100, the server 500 may perform at least some functions of the driving portion 300 of the vehicle 100 instead. The server 500 may process the received data of the vehicle 100 and return or transmit a signal including a control command for controlling the target vehicle 100 to the vehicle 100 based on the processed data. This server 500 may be, for example, a cloud server. The server 500 may be provided by a manufacturer of a vehicle or driver monitoring device/system, but is not limited thereto.

The vehicle 100 may include a smart pad 200, a driving portion 300, a display 400, and the like. In this case, the driving portion 300 may also be referred to as a processor.

The smart pad 200 may be installed on a seat of a vehicle. The smart pad 200 may obtain state information of a vehicle occupant (in particular, a driver) by sensing. Data obtained through the smart pad 200 may be included in the data of the vehicle 100 described above.

The driving portion 300 may process various signals related to driving, such as driving of a vehicle. The driving portion 300 may generate a vehicle control signal based on the driver's condition information obtained through the smart pad 200. Vehicle control signals may include various control signals related to various events in the vehicle, such as control signals related to vehicle driving, control signals not related to vehicle driving but related to measures according to the driver's condition, and information provision control signals through the display 400. In this sense, the driving portion 300 may be referred to as a controller, processor, and the like that controls overall driving, that is, operation of the vehicle, but is not limited thereto.

In FIG. 2, detailed configuration blocks of the vehicle 100 are illustrated.

The driving portion 300 may include a first amplifier (AMP) 310, a filter 320, a comparator 330, a second AMP (340), a converter (350), a processor 360, a battery 370, a transceiver 380, a memory 390, and the like.

However, all of the listed components of the driving portion 300 may not be essential components. According to the embodiment, some of the components listed as components of the driving portion 300 may be omitted. Alternatively, at least one or more components not listed may be added and included in the driving portion 300.

The first AMP 310 may receive and amplify a signal including data sensed by the smart pad 200.

The filter 320 may filter the signal amplified by the first AMP 310.

The comparator 330 may compare the signal filtered by the filter 320 (for convenience, the first signal) and the input reference signal (for convenience, the second signal). The comparator 330 may output the result to the second AMP 340 when the difference between the first signal and the second signal is equal to or greater than a preset threshold as a result of the comparison, and may return the result to the filter 320 otherwise.

In the above, the second signal, that is, the reference signal may include a value set to verify the validity of the data sensed by the smart pad 200 or to process only meaningful sensing data. To this end, the reference signal may be generated based on a value determined by using or referring to a driver's set value or an initial sensed value for the driver.

Meanwhile, the reference signal is not always a fixed value but may vary. For example, even if the vehicle is used every day, the physical condition of the driver may not always be constant or the same. In this case, for example, when boarding a vehicle, a previously set reference and first sensed data of a user may be compared, and then the first sensed data of the user may be used as a reference.

The second AMP 340 may amplify the signal input through the comparator 330 again.

The conversion portion 350 may convert the second amplified signal in the second AMP 340. At this time, conversion may represent, for example, converting an analog signal into a digital signal.

The processor 360 may process a signal based on the signal converted by the conversion portion 350. In connection with signal processing, the processor portion 360 may include at least one or more algorithms related to driver monitoring according to the present disclosure. In other words, the processor 360 may apply an algorithm to the converted signal to process the bio-signal of the driver and analyze the state of the driver. The processor 360 may perform a controller function of controlling the overall operation of the driving portion 300.

The battery 370 may supply power to each component of the driving portion 300 described above. The battery 370 may include software such as algorithms, modules, and programs for controlling power supply to components.

The transceiver 380 may transmit a result processed by the processor 360. The transceiver 380 may receive various signals (including control commands) from the outside and transfer them to the processor 360 to be processed.

The memory 390 may be built into or detachable from/attached to the vehicle 100. The memory 390 may be in the form of a database (DB) server located in a remote location or interlocked therewith.

In the driving portion 300 according to the present disclosure, by processing only meaningful sensing data rather than processing all data sensed by the smart pad 200, it is possible to reduce battery consumption and, to improve data processing speed, and to use efficiently limited resources, compared to processing all data.

In the present disclosure, "monitoring" may indicate, for example, monitoring of a user's bio-signal, but is not limited thereto.

In this regard, examples of technologies for monitoring a user's bio-signals may include photoplethysmography (PPG), electrocardiography (ECG), blood temperature management (BTM), and the like.

PPG is a non-invasive technology that measures blood circulation using infrared light or light and is a technology that measures blood pressure waves generated when blood flows from arteries to the heart to determine pulse rate, heart rate, and heart health status. Such PPG can be easily measured, and is generally used for purposes such as smart watches and smart bands.

ECG is a technology that measures the electrical activity of the heart to determine the state of heart health and generally, electrodes attached to the chest are used to measure the heart's electrical signals, and electrical signals are produced by the nervous system that controls the contraction and relaxation of the heart. These ECGs are used to identify heartbeat rhythms and abnormal signals.

BTM is a technology that controls the user's body temperature, is generally used in heart surgery, critical care, or the like, cools or heats the blood in the body to maintain and stabilize the user's body temperature, so that the user's body temperature is maintained within a certain range, and thus helps reduce the risk of complications. However, these BTMs are generally controlled by medical experts in medical institutions.

Regarding the user monitoring method, monitoring may be performed through a contact method and a non-contact method. However, particularly, in the present disclosure, a technology for monitoring through a non-contact method will be described as an example. However, it is not limited thereto.

The non-contact type monitoring technology according to the embodiment(s) of the present disclosure may use a sensor technology using an electronic yarn. This sensor technology can be applied to various places where the electronic yarn-based electrode pad, that is, the sensor pad described above, can be applied through a method of forming a yarn through a spinning process after manufacturing a core wiring substrate. For example, it may be applied to clothes, shoes, or a watch worn by a user. In addition, when the user is a driver, it may be applied to a seat belt, seat, driver's seat, and the like of a vehicle.

In this way, the monitoring technology through the electronic yarn-based electrode pad can monitor the user's condition in a non-contact manner even if the user (including the driver) does not directly attach or wear it. Vehicle control and provision of various related information may be performed through such monitoring.

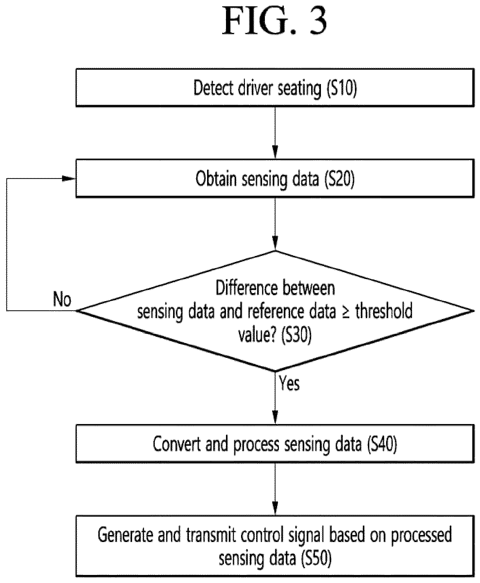

FIG. 3 is a flowchart for explaining a driver monitoring method according to an embodiment of the present disclosure.

Referring to FIG. 3, the driver monitoring device may detect a driver sitting in a seat in the vehicle (S10).

The driver monitoring device may obtain sensing data for the target driver when the driver's sitting on the seat according to getting into the vehicle is detected (S20).

The driver monitoring device may calculate a difference between the obtained sensing data and the reference data, and determine whether the calculated difference is equal to or greater than a preset threshold (S30).

If the calculated difference is less than a preset threshold as a result of the determination in step S30, the driver monitoring device discards or ignores the sensing data obtained in step S20 and waits until new sensing data is obtained. In this regard, sensing data may be continuously obtained for the target driver at predetermined intervals.

The driver monitoring device may convert and process the sensing data obtained in step S20 if the calculated difference is greater than or equal to a preset threshold as a result of the determination in step S30 (S40).

The driver monitoring device may generate and transmit a vehicle control signal based on the sensing data processed in step S40 (S50).

In step S50, the vehicle control signals may include signals not only for controlling the operation of the vehicle and but also signals for controlling other devices in the vehicle, such as heaters, air conditioners, sound devices, and displays. In addition, the vehicle control signal may also include a signal to be transmitted not only to the inside of the vehicle but also to the outside of the vehicle. For example, based on the sensed data, if the driver's physical condition is determined to be in an emergency or very emergency, a rescue signal or the like which is also sent to a pre-registered terminal or a related center (for example, 119 ambulance or the like) can be also included in a vehicle control signal.

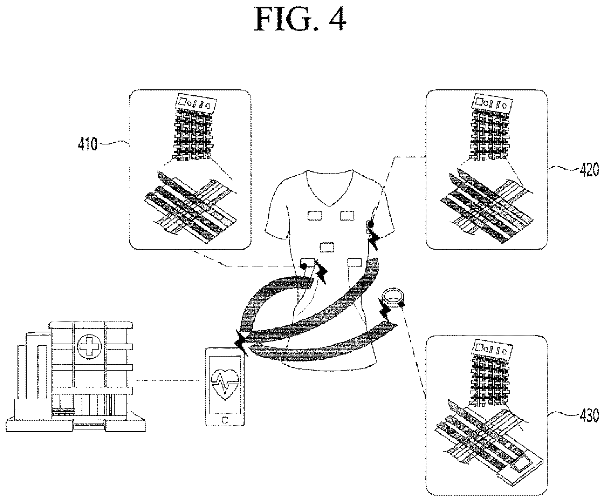

FIG. 4 is a diagram for explaining application of a non-contact monitoring technology according to an embodiment of the present disclosure to clothes and a smart band. However, the corresponding technology is not necessarily applied only to clothes or smart bands, and may be applied, for example, to vehicles.

FIG. 4 illustrates that an ECG monitoring system and a BTM monitoring system are built in clothes, and a PPG monitoring system is built in a smart band. In Figure 4, each system does not have to be built all at once.

As illustrated in FIG. 4, a plurality of monitoring systems may be simultaneously built on clothes or smart bands, or only one monitoring system may be built thereon.

Referring to FIG. 4, the clothes or a smart band 450 may continuously sense a user's state periodically/non-periodically and transmit a related signal to at least one or more terminals through a non-contact monitoring system. In FIG. 4, it is illustrated that a sensing signal is transmitted to a pre-registered terminal of a medical institution or a vehicle (or a controller of a vehicle) via a pre-registered terminal.

At this time, both clothes and smart band illustrated in FIG. 4 may include components such as a battery or an input/output interface for transmitting/receiving signals to a non-contact monitoring system formed through an electronic yarn.

The monitored user's condition may include, for example, an electrocardiogram related to an ECG monitoring system, a body temperature related to a BTM monitoring system, and a pulse wave related to a PPG monitoring system, but is not limited thereto.

Referring to FIG. 4, the electronic yarn-based electrode pad 410 for the ECG monitoring system may be formed by mounting a smart pad sensor on fabric through a sewing process.

The electronic seal-based electrode pad 420 for the BTM monitoring system is also formed by mounting a smart pad sensor on fabric, and the smart pad sensor may use an electrode (Cu), an electrode (Ni/Cu), or the like.

The electronic yarn-based electrode pad 430 for the PPG monitoring system is also formed by mounting a smart pad sensor on fabric, but a light source (eg, R-LED), a sensor (PD: Photodiode), or the like can be used as the smart pad sensor.

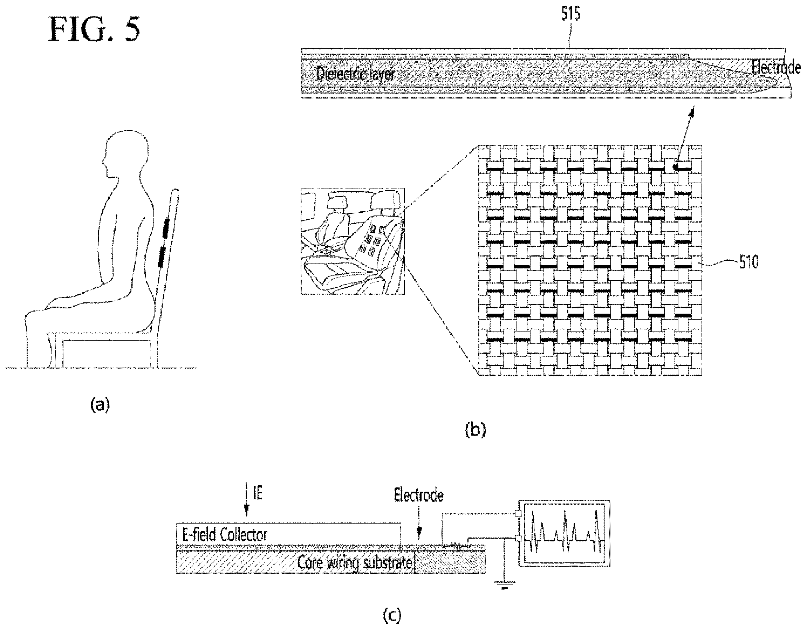

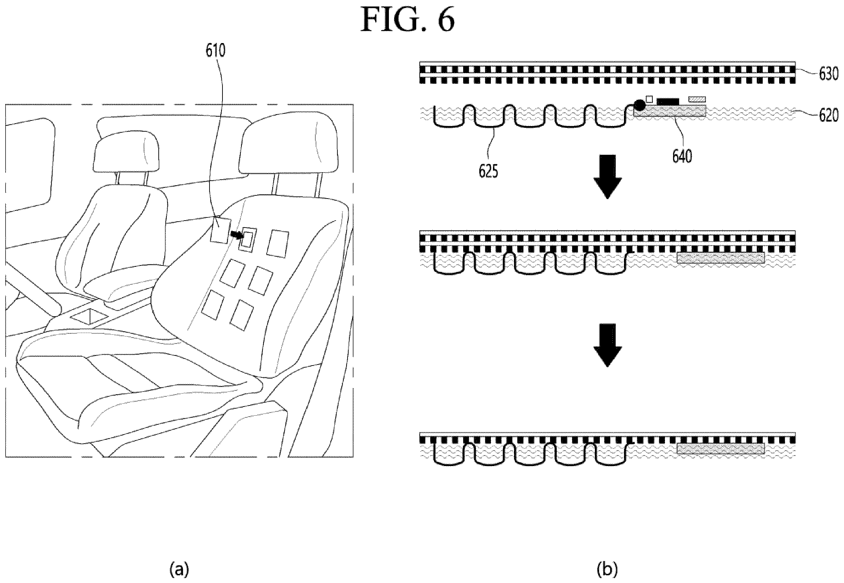

FIGS. 5 and 6 are diagrams for explaining application of a non-contact monitoring technology according to an embodiment of the present disclosure to a vehicle.

In (a) of FIG. 5, the driver monitoring method is explained.

Suppose you want to monitor the driver's heart condition. Referring to (a) of FIG. 5, when a smart pad 200 is built on a seat and a driver sits on the seat, data for a heart condition can be obtained by non-contact sensing.

In this specification, the contact type refers to a case where the smart pad is directly attached to the human body (or skin), otherwise it may be referred to as a non-contact type. In the case of the present disclosure, since the driver's condition is sensed by building the smart pad 200 on a vehicle seat or handle, for example, rather than a method attached to the human body, it can be referred to as a non-contact method. For example, according to the present disclosure, there is no problem in sensing even if a seat cover is covered on a vehicle seat.

Referring to (b) of FIG. 5, a smart pad built into a vehicle seat is illustrated.

In the present disclosure, a smart pad is a fabric-based electrode pad and may be manufactured using a fabric-based electronic yarn structure.

Referring to the enlarged drawing illustrated in (b) of FIG. 5, each smart pad 200 may be implemented in a textile form using an electronic yarn of a predetermined size. However, it is not limited thereto.

The smart pad 200 may include a sensor module. Each sensor constituting the sensor module may include an electronic yarn including a passage through which electrons can move surrounded by an insulating layer. It is preferable that such an electronic yarn is 10 mm or less, but is not necessarily limited thereto.

In (a) to (b) of FIG. 5, the smart pad 200 is expressed for easy explanation, and for example, the smart pad 200 may be embedded without being exposed to the outside at all, or may be difficult to visually distinguish from the outside. To this end, the smart pad 200 may include an indicator (not illustrated) to notify that the smart pad 200 is built or to provide information according to the sensed driver's condition.

According to the present disclosure, in order to monitor the driver's condition in a non-contact manner, the smart pad 200 including the sensor module can be built an electric field collector (E-field collector) on a core substrate as illustrated in (c) of FIG. 5, in order to detect the electric field measured in the sensing process.

FIG. 5 illustrates a smart pad built (or attached) to a vehicle seat through a sawing process, for example, on a predetermined area of the vehicle seat, while FIG. 6 illustrates a detachable/attachable smart pad on a predetermined area of the vehicle seat.

In (a) of FIG. 6, it is illustrated that the smart pad 610 is detachable/attachable.

In (b) of FIG. 6, as illustrated in (a) of FIG. 6, a structure for detaching/attaching a smart pad to/from a vehicle seat is illustrated to explain. However, this is only an example, and the present disclosure is not limited thereto.

In the present disclosure, an adhesive fabric may be used to detach/attach the smart pad to a vehicle seat. However, it is not limited thereto.

Referring to (b) of FIG. 6, an element 640 such as a fabric sensor is mounted on a fabric, and an adhesive fabric layer 630 may be combined on the electronic yarn 620 having one end of electrode pad 625 connected to corresponding element 640. Meanwhile, corresponding to the adhesive fabric layer 630, the vehicle seat may be provided with a corresponding configuration so that the adhesive fabric can be detached/attached so that the smart pad can be easily detached/attached.

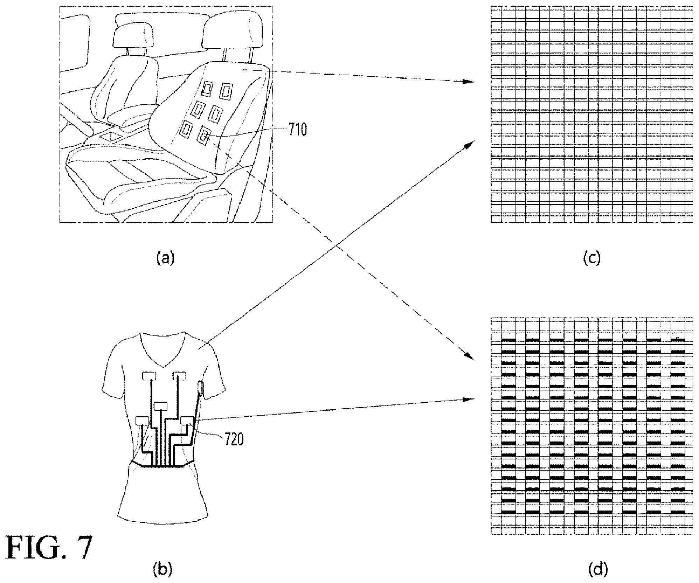

Figure 7 (a) illustrates a smart pad 710 built on a vehicle seat, and Figure 7 (b) illustrates a smart pad 720 built on clothes.

Meanwhile, FIG. 7(c) illustrates a general fiber and FIG. 7(d) illustrates an electronic yarn. Referring to (a) and (b) of FIG. 7, except for the portion of the electronic yarn-based smart pad illustrated in (d) of FIG. 7, other portions may be composed of the general fiber illustrated in (c) of FIG. 7.

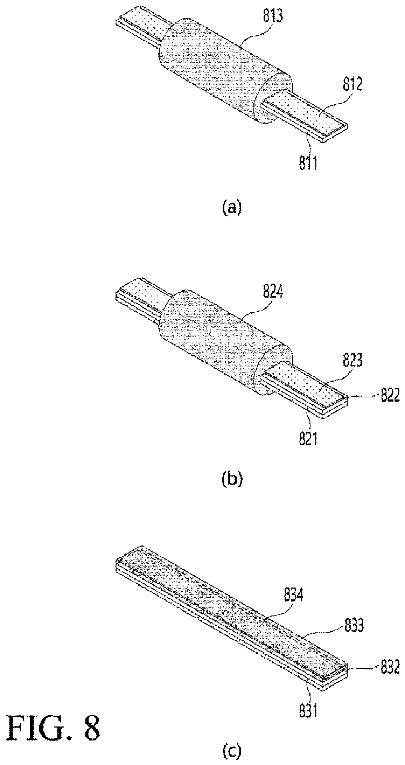

FIGS. 8 to 11 explain the structure of an electronic yarn forming a smart pad.

In the present specification, various types of fibers may be used as the fibers spun on the substrate. For example, such fibers may be various, such as dielectric fibers and polymer-based fibers.

Referring to (a) of FIG. 8, an electronic yarn may be formed by spinning a dielectric fiber 813 on a pure core substrate. In this case, the pure core substrate may be formed by stacking the metal electrode 812 on the PI or GF 811.

Referring to (b) of FIG. 8, an electronic seal may be formed by spinning conductive fibers 824 on a core substrate to which high-strength/elongation fibers are attached. At this time, the core substrate may be formed on the high strength/elongation fiber 821. The core substrate may be the aforementioned pure core substrate. Meanwhile, the high strength/elongation fiber 821 may include nylon or the like.

Referring to (c) of FIG. 8, as in (a) to (b) of FIG. 8, fibers are not spun on the core substrate, but coated to form an electronic yarn. In this case, the core substrate may be the aforementioned pure core substrate. Meanwhile, the core substrate may be formed on the high strength/elongation fiber 831. In addition, the conductive fiber 834 may be applied and coated on the core substrate. In the same manner as described above, the elongation force of the electronic yarn can be enhanced compared to the radiation type. Thereafter, an electronic yarn having a desired size may be obtained by cutting the electronic yarn as needed. However, the thickness of the electronic yarn according to (c) of FIG. 8 is preferably within 50 μm, but is not limited thereto.

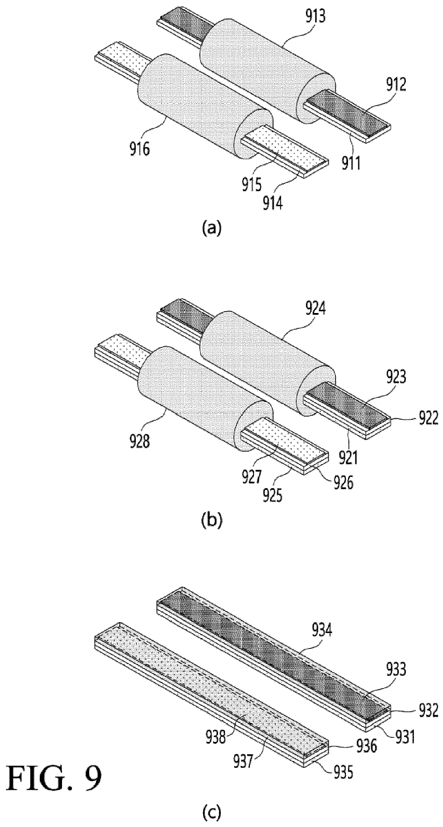

FIG. 9 illustrates, for example, an electronic yarn for temperature measurement, and for this purpose, the electronic yarn may be in the form of a pair. Temperature data can be obtained from the difference in thermal conductivity of the pair-type electronic yarn. For example, a pair may be configured such that one electronic yarn has good thermal conductivity and the other electronic yarn has low thermal conductivity which performs slow heat conduct. At this time, the temperature may be measured by measuring the electromotive force based on the time difference of the heat conduction data obtained through the two electronic yarns.

Referring to (a) of FIG. 9, each electronic yarn constituting a pair may be formed by spinning conductive fibers 913 and 916 on a pure core substrate. However, the structure of each electronic yarn constituting the pair may be different from each other. For example, the pure core substrate of the first electronic yarn includes a metal electrode (Ni/Cu) 913 on the PI or GF 912, and the pure core substrate of the second electronic yarn may include a metal electrode (Cu) 914 on the PI or GF 9124.

Referring to (b) of FIG. 9, in each electronic yarn constituting the pair of (a) of FIG. 9, high-strength/elongation fibers 921 and 925 are attached on the lower end of each core substrate 922-923 and 926-927 and conductive fibers 924 and 928 may be spun to form respective electronic yarns constituting a pair.

Referring to (c) of FIG. 9, each electronic yarn constituting a pair is not formed in a form in which conductive fibers are spun on the core substrate of FIG. 9 (a) or (b), but an electronic yarn may be formed in a form in which conductive fibers are coated. As described above, in this case, at least two electronic yarns forming a pair may be formed through a cutting process, respectively, if necessary.

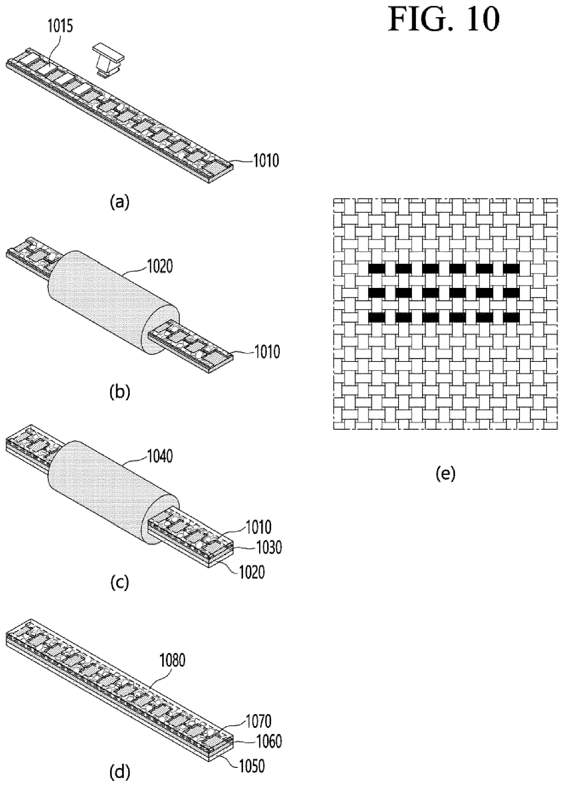

Referring to (a) of FIG. 10, an optical element may be mounted in the electronic yarn. Mounting of this optical element can be performed before the fiber is spun. Meanwhile, the optical element may include LED, ELD, and the like.

For example, green LED PnP 1015 may be mounted on the core substrate 1010 at predetermined intervals.

Referring to (b) of FIG. 10, an electronic seal may be formed by spinning a transparent synthetic fiber 1020 on a core substrate 1020 on which an optical element is mounted.

Referring to (c) of FIG. 10, a core substrate 1030 on which optical elements are mounted is stacked on a high-strength/elongation fiber 1020, a transparent synthetic fiber 1010 is coated on the core substrate 1030, and a transparent synthetic fiber 1040 may be spun on the coating to form an electronic yarn.

Referring to (d) of FIG. 10, it is similar to (c) of FIG. 10, but a core substrate 1060 on which an optical element 1070 is mounted is stacked on a high-strength/elongation fiber 1050, and the transparent synthetic fiber 1080 is coated on the core substrate 1060 to form an electronic yarn.

When the electronic yarn formed in the same manner as in (b) to (d) of FIG. 10 is mounted in a sawing process between fabrics as illustrated in (e) of FIG. 10, the electronic yarn containing the optical element, that is, the green light fabric electronic yarn can be externally identified through light emission.

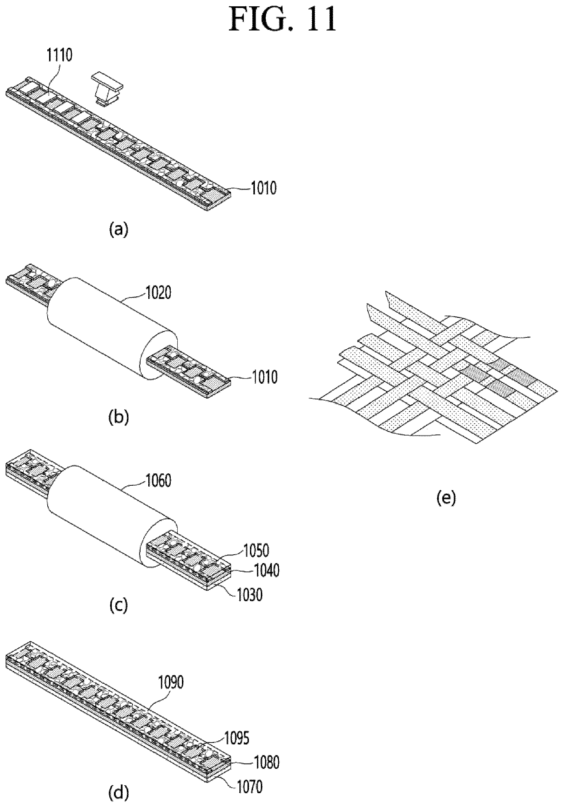

Referring to (a) of FIG. 11, unlike (a) of FIG. 10, instead of mounting an optical element, a general element may be mounted in an electronic yarn. These general elements may include batteries, sensors, and the like.

In other words, the electronic yarn can be formed by, for example, mounting a general element PnP on a core substrate and spinning synthetic fibers thereon.

(b) to (d) of FIG. 11 are the same except that general elements are mounted on the core substrate instead of optical elements, so refer to the above description.

When a smart pad is formed through the electronic yarn as illustrated in (b) to (d) of FIG. 11, a fabric-based wearable battery may be formed as illustrated in (e) of FIG. 11.

The synthetic fiber of FIG. 11 may be replaced with the aforementioned transparent synthetic fiber or polymer-based fiber.

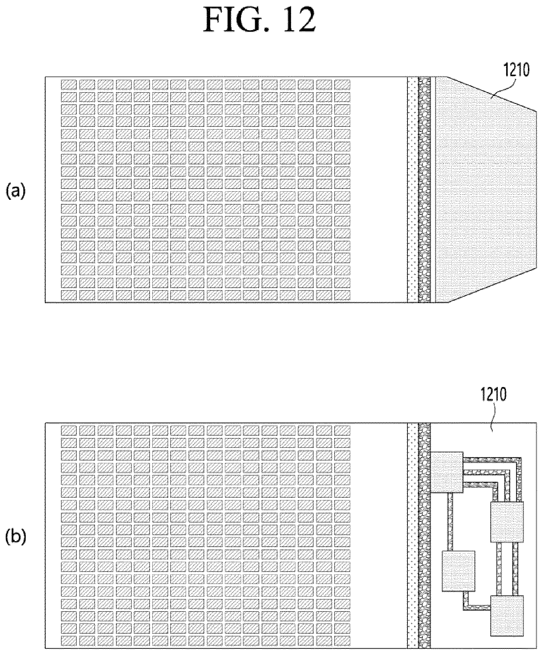

FIG. 12 illustrates a driving portion assembled with a smart pad. In this case, a monitoring system based on at least one of ECG, BTM, and PPG may be disposed in the driving portions 1210 and 1220.

Referring to (a) of FIG. 12, as a smart pad itself, a driving portion 1210 may be assembled to one end of the smart pad to form a monitoring system.

Referring to (b) of FIG. 12 also, as a smart pad itself, the driving portion 1220 may be mounted on one area of the smart pad and then assembled to form a monitoring system.

However, in (a) of FIG. 12, the entire driving portion configures one monitoring system, whereas in (b) of FIG. 12, the monitoring system may be segmented and disposed in the driving portion.

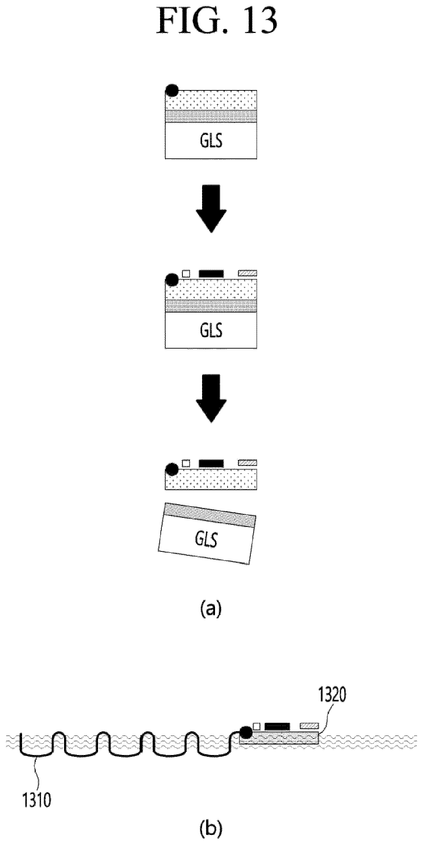

FIG. 13 is a diagram for explaining a method of directly mounting a driving board on fabric. In other words, when a fabric-based monitoring system is built, a board is placed on the fabric.

Referring to (a) of FIG. 13, a wiring layer may be formed by first stacking a sacrificial layer on a glass substrate GLS, forming a PI or GF substrate thereon. Thereafter, components may be mounted, and a driving board may be formed by separating the glass substrate GLS after mounting.

Referring to (b) of FIG. 13, a driving board (for example, a fiber sensor) may be directly mounted on and attached to a fabric and connected through an electronic yarn.

After mounting, you can communicate with the wireless signal transmission board to check if the mounting was successful.

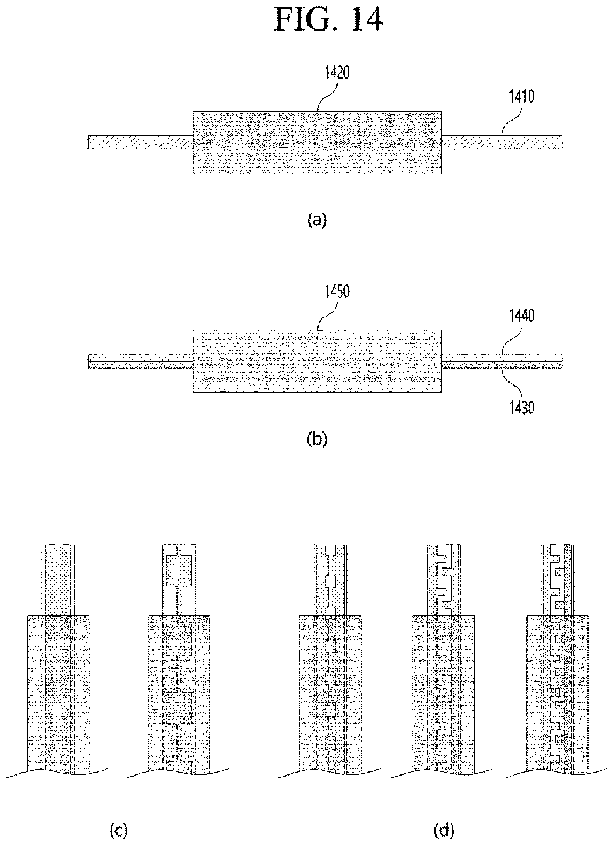

FIG. 14 is a diagram for explaining the formation of the electronic yarn through a method of forming a yarn through a spinning process after manufacturing a core wiring substrate.

In (a) of FIG. 14, an electronic yarn is formed by spinning a conductive fiber 1420 on a general fiber 1410. However, through these electronic yarns, only electrical signals are transmitted.

Meanwhile, in (b) to (d) of FIG. 14, an electronic yarn according to the present disclosure is illustrated.

Basically, referring to (b) of FIG. 14, an electronic yarn may be formed by spinning a fiber 1450 on a PI or GF-based core substrate 1430 on which a metal electrode 1440 is formed.

At this time, the fiber to be spun may be a conductive fiber as illustrated in (c) of FIG. 14 or a general fiber as illustrated in (d) of FIG. 14.

Referring to (c) and (d) of FIG. 14, metal electrodes having various electrode patterns may be formed on the substrate.

Meanwhile, it is preferable that both the width and thickness of the electronic yarn according to the present disclosure are 5 mm or less, but are not limited thereto.

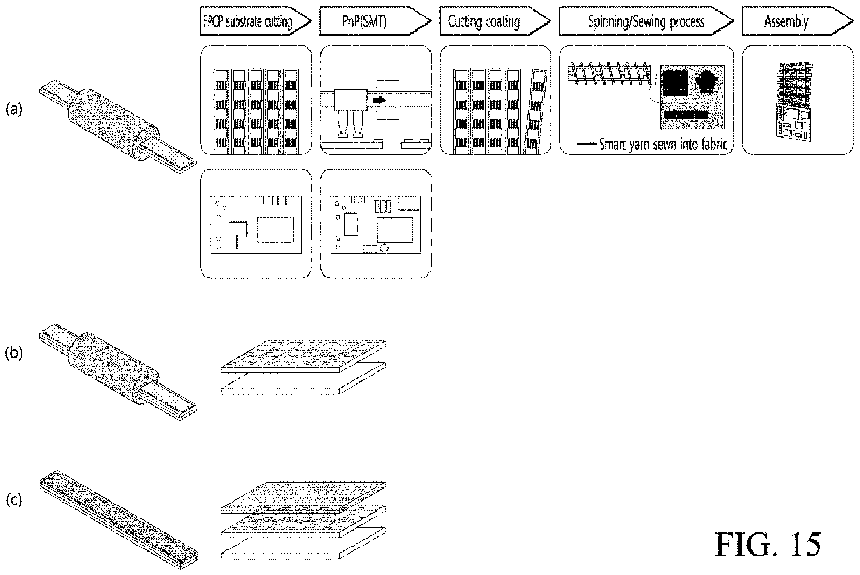

FIG. 15 is a diagram for explaining a method of manufacturing an electronic yarn used as a smart pad (that is, an electrode pad).

In (a) of FIG. 15, for convenience, the electronic yarn is formed by using a conductive fiber on a pure core board as an example.

First, the FPCB substrate may be cut, and PnP may be formed through an SMT process.

Thereafter, the FPCB substrate is cut as desired, and a fiber yarn is spun on the cut FPCB substrate, and spinning and sewing processes are performed to form an electronic yarn and an electrode pad using the same.

Then, by assembling the driving board and the pad, an electronic yarn-based smart pad can be formed.

Referring to (b) of FIG. 15, an electronic yarn may be formed by stacking a core substrate on a high-strength/elongation fiber and then spinning a conductive fiber thereon. In this case, in particular, a process of attaching to the fiber fabric may be further inserted in the above process. In other words, a process of attaching high-strength/elongation fibers to the fiber fabric is added to form an electronic yarn, through which an electrode pad can be formed.

Referring to (c) of FIG. 15, an electronic yarn may be formed by stacking a core substrate on high-strength/elongation and conductive fibers and coating the conductive fibers on the core substrate.

In (c) of FIG. 15, although a fiber fabric attachment process is added in the above process, the sewing process is directly performed except for the spinning process in the above process to form an electronic yarn, and an electrode pad can be formed using the same. Since the conductive fibers are attached to the core substrate rather than spinning the conductive fibers, the spinning process can be omitted.

Meanwhile, the process of FIG. 15 may be referred to in a similar manner even when an optical element is mounted or a non-optical element (sensor, battery, or the like) is mounted.

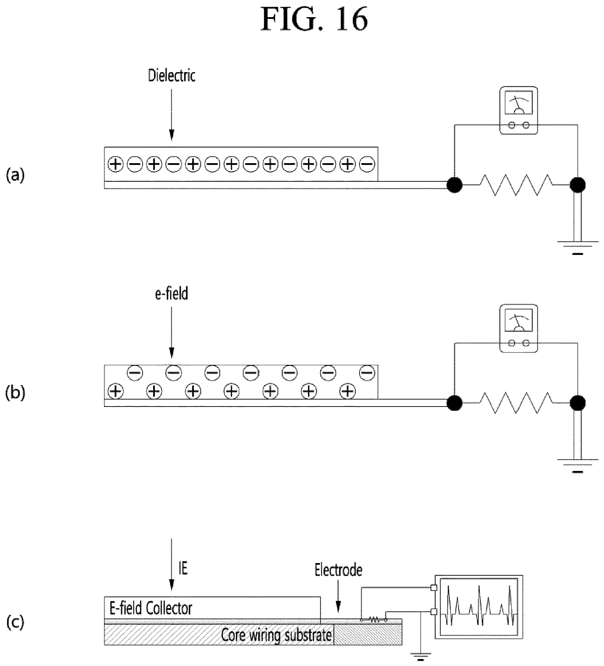

In FIG. 16 and below, in order to build a non-contact type monitoring system according to the present disclosure, an E-field collector for detecting an electric field in an electronic yarn may be built on a core substrate.

As illustrated in (a) of FIG. 16, the dielectric may be coated on the core wiring substrate, and the arrangement may be changed by applying an electric field as illustrated in (b) of FIG. 16.

Therefore, as illustrated in (c) of FIG. 16, an electrode may be stacked on the core wiring substrate, and an electric field collector may be formed thereon.

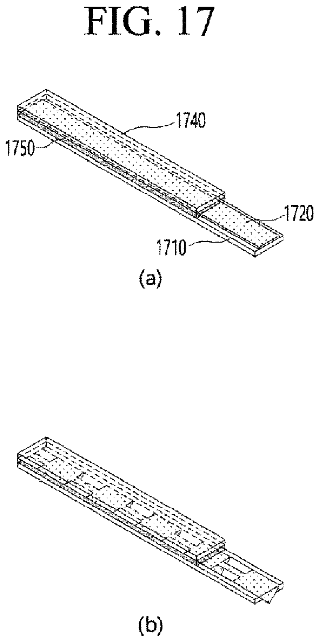

In (a) of FIG. 17, for example, an electrode 1720 is stacked on a core substrate 1710 such as PI or GF, a dielectric layer 1730 is stacked on the electrode 1720, and a protective film 1740 can be stacked thereon, eventually forming an electric field collector. In this case, the protective film 1740 may include glass fibers, but is not limited thereto.

(b) of FIG. 17 is similar to (a) of FIG. 17, but (b) of FIG. 17 may be different from (a) of FIG. 17 in that the electrodes 1720 are not continuously stacked on the core substrate 1710, but the electrodes 1720 are arranged at predetermined intervals and disconnected.

In the present specification, various fibers may include cotton, silk, wool, rayon, acetate, nylon, polyester, acrylic, and the like. An appropriate fiber may be selected with reference to at least one of strength, wet strength, elongation (%), and the like of each fiber.

Following the non-contact monitoring system described above, the contact monitoring system will be described in more detail. At this time, even if it is a contact type, the overlapping or repeated description of the above content may be omitted and the same may be used or applied mutatis mutandis.

As an example of such a contact monitoring system, a PPG monitoring method will be described below as an example.

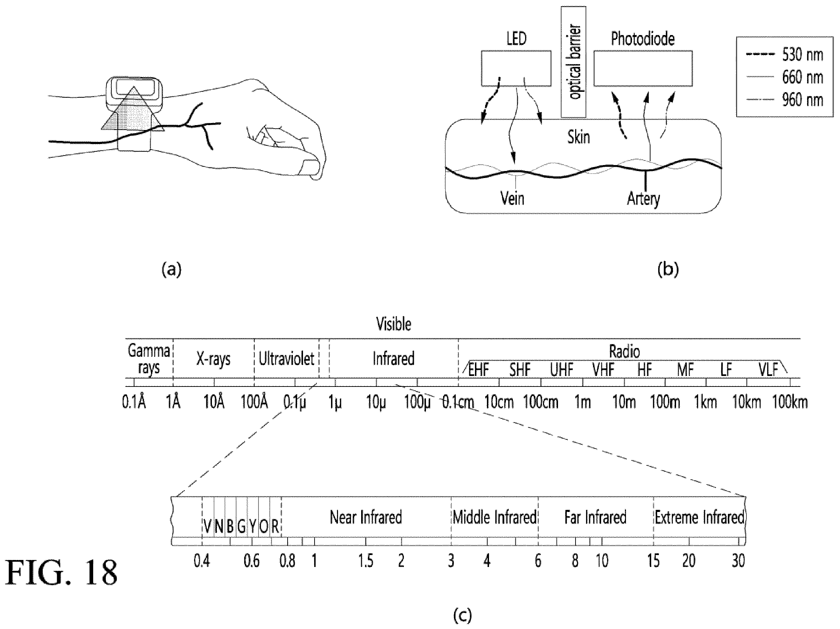

In FIG. 18, the appearance of wearing the PPG sensor and principle thereof will be described.

Referring to (a) of FIG. 18, a state of wearing a smart pad equipped with a ppg sensor on a wrist is illustrated.

Referring to (b) of FIG. 18, a structure in which, when light is irradiated into the skin in the PPG sensor in which an optical element (for example, LED) is built through this, the light reflected from the blood vessel is received by a photodiode (PD) is provided. At this time, an optical barrier is built between the optical element and the PD so that incident light does not interfere with the reflected light.

Meanwhile, in (c) of FIG. 18, a wavelength band mainly used by the PPG sensor, that is, a region is illustrated. For example, the PPG sensor may mainly use wavelength bands such as 530 nm, 660 nm, and 940 nm, but is not limited thereto.

In the present disclosure, in DMS using a fabric electronic yarn-based PPG sensor, the contact PPG sensor may have ethanol reflectance spectrum characteristics for detecting ethanol-absorbed blood.

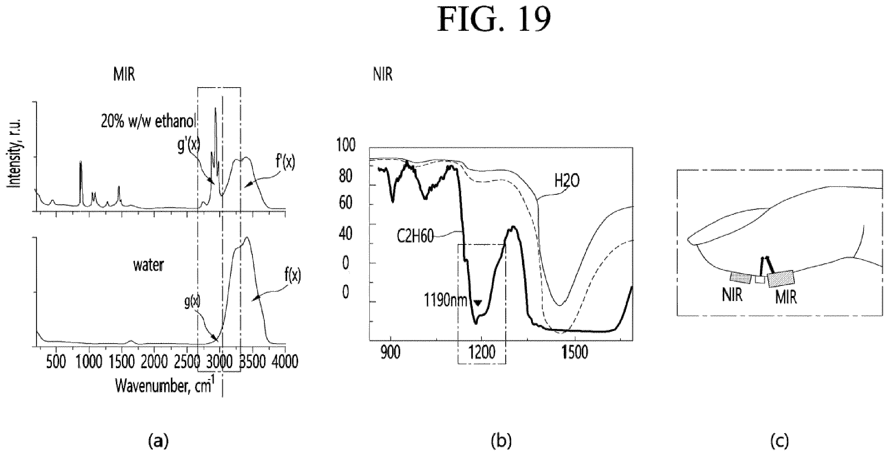

As illustrated in (c) of FIG. 19, the MIR and NIR sensors can be operated according to the contact area and method of the finger.

(a) of FIG. 19 illustrates the MIR ethanol reflectance spectrum for detecting ethanol-absorbed blood. Unlike water, 20% ethanol can have a significant value between 2700 and 3100 nm. In particular, it may be a peak wavelength at 2890 nm. In other words, if the peak wavelength related to the drinking measurement is 2890 nm, the oxygen saturation may be 660 or 940 nm, and the pulse wave may be 530 or 660 nm. However, the present disclosure is not limited thereto. This can be modified by conditions or various factors.

On the other hand, in (b) of FIG. 19, the NIR ethanol reflection spectrum for detecting ethanol-absorbed blood is illustrated, and referring to the NIR transmission spectrum, between 1100 and 1300 nm, ethanol can have a significant value completely different from that of water.

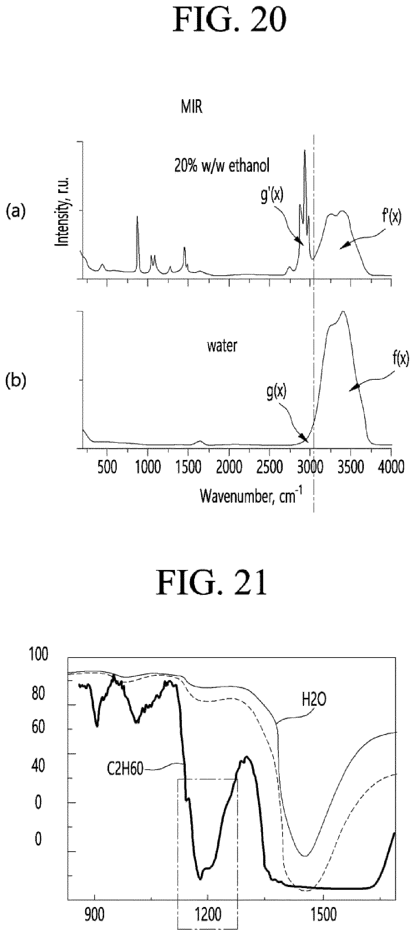

FIG. 20 relates to the MIR transmission spectrum of (a) of FIG. 19, and the g'(x) value of ethanol compared to g(x) of water and the f(x) value of ethanol compared to f(x) of water are described.

Equations 1 and 2 below may be referred to.

【Equation 1】

$$\left(\left(1-\frac{SI_{MIR}-RI_{MIR}}{SI_{MIR}}\right)+\left(1-\frac{SI_{MIR}-RI_{MIR}}{SI_{MIR}}\right)\right)\times 100$$

【Equation 2】

$$\int_{2700}^{3100} g'(x)dx+\int_{2700}^{3100} f'(x)dx-\left(\int_{2700}^{3100} g(x)dx+\int_{2700}^{3100} f(x)dx\right)$$

In the above, SI may represent reflected light intensity of sample, and RI may represent reflected light intensity of reference sample. However, it is not limited thereto.

Therefore, blood absorbing ethanol can be irradiated using the principle that ethanol reflects and water transmits at the corresponding wavelength, and through this, it is possible to determine whether the user, that is, the driver, is drinking. The data on the presence or absence of alcohol determined in this way may be used for various vehicle control such as processing of vehicle operation failure and transmission of the contents to the terminal.

In FIG. 21, like FIG. 20, contents relating to the NIR transmission spectrum illustrated in (b) of FIG. 19 is provided. In this case, Equation 3 may be referred to.

【Equation 3】

$$\int_{1100}^{1300} g_{SI}(x)dx-\int_{1100}^{1300} g_{RI}(x)dx$$

In the above, SI may represent reflected light intensity of sample, and RI may represent reflected light intensity of reference sample. However, it is not limited thereto.

According to the present disclosure, an example in which an electronic yarn-based PPG sensor is mounted on a handle of a vehicle is illustrated.

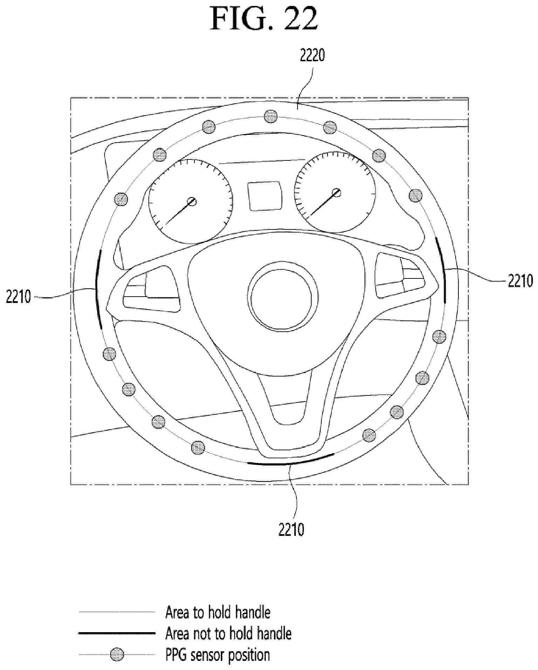

Referring to FIG. 22, for example, the circumference of the steering wheel is about 120 cm, the diameter thereof is 300 mm and the steering wheel is based on 3 frames. However, this is only an example and is not limited thereto.

Meanwhile, the smart fabric electronic yarn may be largely divided into an area 2210 where the driver holds the handle and an area 2220 where the driver cannot hold the handle in relation to each frame.

Referring to FIG. 22, PPG sensors may be disposed at predetermined intervals on a handle holding area 2210. However, this is only an example, and the disposition interval, disposition shape, or the like of the sensors may vary. In addition, although the sensor is illustrated as being disposed only on the upper portion of the handle, it may be mounted on the rear side as well, and may be alternately mounted on the front and rear surfaces.

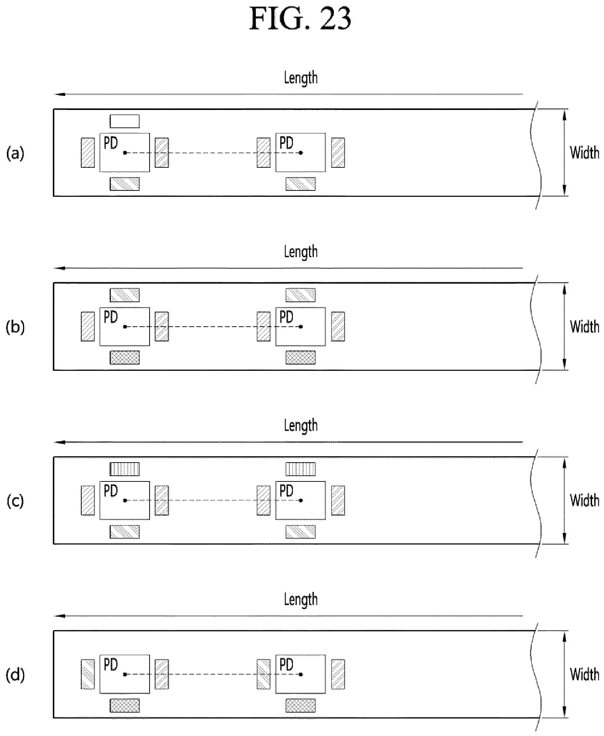

In FIG. 23, it is possible to wire bond each sensor discontinuously disposed on the handle, which is illustrated to explain wire bonding conditions.

At this time, gender or the like may be referred to as considerations related to sensor placement and bonding. The average male hand has a height of 189 mm and a width of 84 mm, and the average female hand has a height of 172 mm and a width of 74 mm. However, this may be defined differently according to various circumstances such as country and region.

Referring to (a) of FIG. 23, one ppg sensor may include a PD, an MIR sensor, a NIR sensor, and a light receiving portion.

It is preferable that each PPG sensor be disposed at an interval of at least 10 cm or more, but this is only an example, and is not limited thereto.

In (b) to (d) of FIG. 23, various embodiments of the configuration of the ppg sensor are illustrated.

In (b) of FIG. 23, the ppg sensor may include a plurality of light receiving portions having different wavelengths from the PD, MIR sensors, and NIR sensors.

In (c) of FIG. 23, the ppg sensor may include a plurality of light receiving portions having different wavelengths from the PD, MIR sensors, and NIR sensors.

In (d) of FIG. 23, the ppg sensor may include a plurality of light receiving portions having different wavelengths from the PD sensors, and IR sensors.

Meanwhile, although not illustrated, the configuration of each ppg sensor built into one handle may be different from each other by combining the configurations illustrated in FIG. 23 with each other.

In addition, in the case of the configurations of (a) to (c) of FIG. 23, different disposition intervals may be applied.

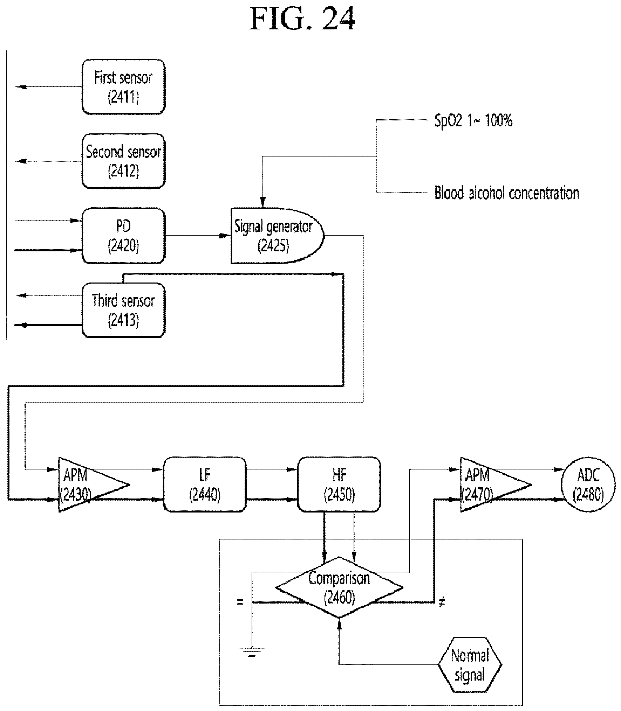

FIG. 24 is a configuration block diagram of a driver monitoring system including a PPG sensor formed based on an electronic yarn according to an embodiment of the present disclosure.

At this time, the same function as the configuration described in FIG. 2 may refer to FIG. 2.

The sensor module may include a first sensor 2411, a second sensor 2412, a third sensor 2413, a photodiode 2420, and a signal generator 2425.

The first sensor 2411 is a light sensor of a first wavelength (950 nm) and may sense oxygen saturation.

The second sensor 2412 is a light sensor of a second wavelength (880-890 nm) and may sense the ethanol concentration.

The third sensor 2413 is a light sensor of a third wavelength (660 nm) and may sense a pulse wave.

The photodiode 2420 receives light reflected according to the light emitted from each sensor, and the signal generator 2425 may generate and transmit a signal based on the light received by the photodiode 2420.

The signal generated and transmitted by the signal generator 2425 may be input to the comparator 2460 through an amplifier (AMP) 2430, a low pass filter (LF) 2440 and a high pass filter (HF) 2450.

The comparator 2460 compares a normal signal (that is, a reference signal) with an input signal to determine whether the signal has been processed. Through this, the monitoring system does not process all sensing data, but processes meaningful data, that is, data only when the condition abnormality of the driver is monitored, so that resources can be efficiently used, power consumption can be reduced, and signal processing speed can be improved. Condition abnormality may indicate, for example, a case where an ethanol concentration equal to or higher than a predetermined threshold value is detected.

In the comparator 2460, among the data collected through monitoring, data without problems such as health and safety are skipped through the comparison group, and normal data is managed only during the monitored time, thereby significantly reducing the amount of data to be processed.

The signal passing through the comparator 2460 is input to the conversion portion 2480 through the amplifier 2470 and converted into a digital signal, and the controller (not illustrated) may generate a vehicle control signal. The signal generated in this way may be transmitted and outputted to a display or terminal in the vehicle, or may be immediately used for vehicle control.

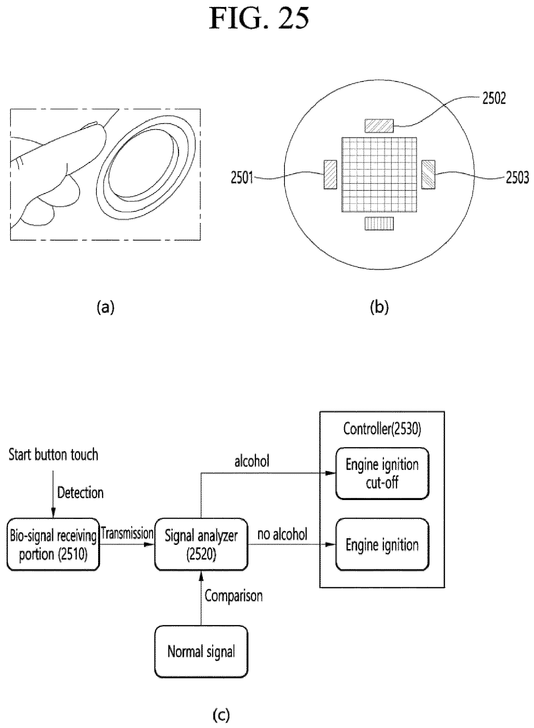

FIG. 25 is an embodiment referring to a button provided in a vehicle, rather than a handle of FIG. 18 and below described above. For convenience of explanation, the button is one example of a start button, but is not necessarily limited thereto. In addition, in addition to the in-vehicle button, it can be applied in a similar manner to the door side of the vehicle or to the remote start button.

In (a) of FIG. 25, the start button is illustrated, and in (b) of FIG. 25, the configuration of the PPG sensor provided in the start button is illustrated. At this time, the PPG sensor may be provided with a plurality of optical wavelength sensors, for example, as described above, a first sensor with a wavelength of 980 nm for measuring the concentration of oxygen saturation, a second sensor with a wavelength of 890 nm for measuring the concentration of ethanol, and a third sensor having a wavelength of 660 nm for pulse wave measurement may be disposed.

In (b) of FIG. 25, the first to third sensors 2501 to 2503 may be disposed at predetermined intervals and areas, but this is only an example and is not limited thereto. Meanwhile, the number or disposition of sensors may be different from those illustrated, according to the purpose or the like.

In relation to this, in (c) of FIG. 25, when the PPG sensor is provided on a button such as a start button, not on a handle, the processing configuration is illustrated.

The processing component may include a bio-signal receiver 2510, a signal analyzer 2520, a controller 2530, and the like.

The bio-signal receiving portion 2510 may receive the driver's bio-signal sensed through each of the sensors when the driver touches the start button. The driver bio-signal received may be a signal transmitted by the signal generator 2425 after receiving a signal of a specific wavelength transmitted to each sensor from the above-described PD 2420 of FIG. 24.

The signal analyzer 2520 may determine whether the driver's bio-signal received through the bio-signal receiver 2510 has been processed.

The signal analyzer 2520 may determine whether the driver has been drinking by comparing the received bio-signal of the driver with the reference signal. The reference signal may be a signal in which a bio-signal is pre-registered when not drinking, that is, when there is no alcohol (ethanol).

The controller 2530 may control the vehicle based on the analysis result of the signal analyzer 2520. At this time, for convenience, in FIG. 25, vehicle control is described as ignition or ignition cut-off of an engine for vehicle operation, but is not limited thereto.

If alcohol is not detected as a result of analysis by the signal analyzer 2520, the controller 2530 may control an engine for driving the vehicle to be ignited. However, if alcohol is detected as a result of analysis by the signal analyzer 2520, the controller 2530 may control the engine for driving the vehicle not to be ignited, that is, the engine ignition to be blocked.

When alcohol is detected according to the analysis result of the signal analyzer 2520, the controller 2530 performs verification multiple times and when the same result is continuously obtained, the controller 2530 can notify the fact through the terminal or perform a separate vehicle control operation. The separate vehicle control operation may control an operation that minimizes vehicle battery consumption so that the vehicle start button is continuously pressed and the battery is consumed to prevent the vehicle from becoming completely inoperable. At this time, the vehicle start button may not be pushed at all (that is, not activated even when touched). Through the in-vehicle display or speaker, the driver can be notified of the impossibility of driving and the reason for the impossibility. Alternatively, when the corresponding fact is notified to the terminal, the location information of the corresponding vehicle may also be transmitted.

The above-described PPG sensor provided on the handle or the start button of a vehicle may be formed using the electronic yarn configured or composed as described above.

Meanwhile, in the case where a plurality of sensors are built in the present disclosure, a method of activating other sensors to perform related operations and controls may be implemented only when an analysis result based on sensing data of at least one sensor is determined to be equal to or greater than a threshold value (status abnormality).

The controller may monitor whether an access signal is sensed in the vehicle. As a result of monitoring, the controller generates and transmits a vehicle control signal when sensing data is not obtained for a predefined time from at least one contact sensor disposed on the handle of the vehicle at predetermined intervals even though an access signal is sensed in the vehicle.

In this case, the PPG sensor may not be disposed on the frame extension portion of the handle illustrated in FIG. 22, and in this case, if the driver is touching only the portion, data for determining whether the driver is drinking may not be obtained. However, if a separate access signal is detected in the vehicle, since the driver exists and is doing something to try to control the vehicle, measures can be taken to operate the vehicle only when the driver's state monitoring accurately detects whether or not he or she is drunk.

Meanwhile, in the present disclosure, a combination of the sensor provided in the above-described vehicle seat, the sensor provided in the handle, the start button, or other sensors provided in a predetermined configuration in the vehicle is used, and when any one sensor data is meaningful, the sensor date is used and thus the driver's condition can be more accurately monitored through other sensors and the vehicle can be controlled accordingly.

Although the present disclosure has described the monitoring technology for the driver in the vehicle for convenience of description, it will be obvious to those skilled in the art that it can be applied to various devices or systems other than the vehicle.

Alternatively, the monitoring device according to the present disclosure may be applied to human clothes.

Even if not specifically mentioned, the order of at least some of the operations disclosed in this disclosure may be performed simultaneously or in a different order from the previously described order, or some may be omitted/added.

According to one embodiment of the present disclosure, the above-described method can be implemented as a processor-readable code in a medium on which a program is recorded. Examples of media readable by the processor include ROM, RAM, CD-ROM, magnetic tape, floppy disk, optical data storage device, and the like.

The display device described above is not limited to the configuration and method of the above-described embodiments, but the above embodiments may be configured by selectively combining all or part of each embodiment so that various modifications can be made.


Claims

1. A method for controlling a driver monitoring system, the method comprising:

detecting a user;
obtaining sensing data for the detected user;
converting the sensing data when a difference between the sensing data and predetermined reference data is equal to or greater than a threshold value; and
generating and transmitting a first vehicle control signal based on the converted sensing data,
wherein the sensing data is obtained through at least one contact sensor provided in the vehicle.

2.  The method of claim 1, wherein the at least one contact sensor is formed using a fabric electronic yarn.

3.  The method of claim 2, wherein the at least one contact sensor is built on a handle of the vehicle.

4.  The method of claim 3, wherein the at least one contact sensor is disposed on the handle of the vehicle at predetermined intervals and disposed in an area other than the extended portion of a frame of the handle.

5.  The method of claim 3, wherein the at least one contact sensor includes of a plurality of sensors, light receivers, and signal generators.

6.  The method of claim 5, wherein the plurality of sensors includes one Mid-Infrared, MIR, sensor, one Near-Infrared, NIR, sensor, and a sensor generating a first wavelength.

7.  The method of claim 5, wherein the plurality of sensors includes one MIR sensor, one NIR sensor, a sensor generating a first wavelength, and a sensor generating a second wavelength.

8.  The method of claim 5, wherein the plurality of sensors includes one IR sensor, a sensor generating a first wavelength and a sensor generating a second wavelength.

9.  The method of claim 4, wherein the contact sensor disposed on the handle has different configurations and placement intervals of each sensor according to a placement area.

10. The method of claim 5, wherein the plurality of sensors includes at least one of a first sensor of a first wavelength for measuring oxygen saturation, a second sensor of a second wavelength for measuring ethanol concentration, and a third sensor of a third wavelength for measuring pulse wave.

11. The method of claim 4, further comprising:
    monitoring whether an access signal is sensed in the vehicle.

12. The method of claim 11, further comprising:
    as a result of the monitoring, when an access signal is sensed in the vehicle, and sensing data is not obtained for a predefined time from at least one contact sensor disposed on the handle of the vehicle at predetermined intervals, generating and transmitting a second vehicle control signal.

13. The method of claim 2, wherein the at least one contact sensor is built in at least one of a start button of the vehicle, a button built in the vehicle, or a button on the vehicle remote control device.

14. The method of claim 13, wherein the at least one contact sensor includes at least one of a first sensor of a first wavelength for measuring oxygen saturation, a second sensor of a second wavelength for measuring ethanol concentration, or a third sensor of a third wavelength for measuring pulse wave.

15. An apparatus for controlling a driver monitoring system comprising:

    a sensor portion configured to detect a user and obtains sensing data for the detected user; and
    a processor configured to, when the difference between the sensing data and predetermined reference data is equal to or greater than a threshold value, convert the sensing data and generate and transmit a vehicle control signal based on the converted sensing data,
    wherein the sensing data is obtained through at least one contact sensor provided in the vehicle.

# FIG. 1

<u>1</u>

# FIG. 2

EP 4 470 463 A1

# FIG. 3

Detect driver seating (S10)

Obtain sensing data (S20)

Difference between sensing data and reference data ≥ threshold value? (S30)

No

Yes

Convert and process sensing data (S40)

Generate and transmit control signal based on processed sensing data (S50)

# FIG. 4

FIG. 5

515

Dielectric layer                                                                Electrode

510

(a)

(b)

IE

Electrode

E-field Collector

Core wiring substrate

(c)

EP 4 470 463 A1

# FIG. 6

(a)

(b)

EP 4 470 463 A1

FIG. 7

813
812
811

(a)

824
823
822
821

(b)

834
833
832
831

FIG. 8

(c)

(a)

(b)

FIG. 9

(c)

# FIG. 10

(a)

(b)

(c)

(d)

(e)

# FIG. 11

(a)

(b)

(c)

(d)

(e)

# FIG. 12

(a)

1210

(b)

1210

# FIG. 13

(a)

1320

1310

(b)

# FIG. 14

(a)

(b)

(c)                              (d)

FIG. 15

EP 4 470 463 A1

# FIG. 16

Dielectric

(a)

e-field

(b)

IE          Electrode

E-field Collector

Core wiring substrate

(c)

29

# FIG. 17

(a)

(b)

LED    optical barrier    Photodiode

---- 530 nm
—— 660 nm
—·— 960 nm

Skin

Vein    Artery

(a)    (b)

Visible

| Gamma rays | X-rays | Ultraviolet | Infrared | | Radio | | | | | | | | |
|---|---|---|---|---|---|---|---|---|---|---|---|---|---|
| | | | | | EHF | SHF | UHF | VHF | HF | MF | LF | VLF | |

0.1Å  1Å  10Å  100Å  0.1μ  1μ  10μ  100μ  0.1cm  10cm  100cm  1m  10m  100m  1km  10km  100km

| V N B G Y O R | Near Infrared | Middle Infrared | Far Infrared | Extreme Infrared |
|---|---|---|---|---|

0.4    0.6  0.8  1    1.5    2    3    4    6    8  10    15    20    30

FIG. 18

(c)

# FIG. 19

(a)

(b)

(c)

# FIG. 20

MIR

(a) 20% w/w ethanol
g'(x)
f'(x)

(b) water
g(x)
f(x)

Wavenumber, cm$^{-1}$

# FIG. 21

H2O

C2H6O

# FIG. 22

Area to hold handle
Area not to hold handle
PPG sensor position

# FIG. 23

# FIG. 24

First sensor
(2411)

Second sensor
(2412)

PD
(2420)

Signal generator
(2425)

SpO2 1~ 100%

Blood alcohol concentration

Third sensor
(2413)

APM
(2430)

LF
(2440)

HF
(2450)

APM
(2470)

ADC
(2480)

Comparison
(2460)

= 

≠

Normal
signal

# FIG. 25

(a)

2501    2502    2503

(b)

Start button touch

Detection

Bio-signal receiving portion (2510) —Transmission→ Signal analyzer (2520)

alcohol → Controller(2530)

Engine ignition cut-off

no alcohol → Engine ignition

Comparison

Normal signal

(c)

Europäisches
Patentamt

European
Patent Office

Office européen
des brevets

# EUROPEAN SEARCH REPORT

**Application Number**

EP 24 17 8768

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/000397 A1 (SHAM WELLEN [TW]) 4 January 2018 (2018-01-04) * paragraph [0027] * * paragraph [0040] - paragraph [0047] * * figure 2 * | 1-15 | INV. A61B5/1455 A61B5/024 A61B5/18 A61B5/00 |
| Y | US 2004/022393 A1 (JONES MARCUS RICHARD [GB]) 5 February 2004 (2004-02-05) * paragraph [0010] * * paragraph [0038] - paragraph [0040] * | 1-15 | |
| A | KR 2022 0132108 A (UNIV SOONCHUNHYANG IND ACAD COOP FOUND [KR]) 30 September 2022 (2022-09-30) * paragraph [0025] - paragraph [0028] * * figure 1 * | 1-15 | |
| A | CN 206 797 260 U (BEIJING SINCODEST ELECTRONIC TECH RESEARCH INSTITUTE) 26 December 2017 (2017-12-26) * paragraph [0008] * * paragraph [0037] - paragraph [0038] * | 1-15 | |
| A | EP 3 505 051 A1 (SANMINA CORP [US]) 3 July 2019 (2019-07-03) * paragraph [0039] * * paragraph [0118] * | 1-15 | **TECHNICAL FIELDS SEARCHED (IPC)** A61B |

The present search report has been drawn up for all claims

| Place of search | Date of completion of the search | Examiner |
|---|---|---|
| The Hague | 16 October 2024 | Costa Angeli, M |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

........................................................................

& : member of the same patent family, corresponding
 document

EPO FORM 1503 03.82 (P04C01)

## ANNEX TO THE EUROPEAN SEARCH REPORT
## ON EUROPEAN PATENT APPLICATION NO.

EP 24 17 8768

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2024

| Patent document cited in search report | | Publication date | Patent family member(s) | | | Publication date |
|---|---|---|---|---|---|---|
| US 2018000397 | A1 | 04-01-2018 | CN | 106889983 | A | 27-06-2017 |
| | | | CN | 207940895 | U | 09-10-2018 |
| | | | EP | 3264382 | A1 | 03-01-2018 |
| | | | HK | 1246490 | A1 | 07-09-2018 |
| | | | US | 2018000397 | A1 | 04-01-2018 |
| US 2004022393 | A1 | 05-02-2004 | DE | 10325430 | A1 | 05-02-2004 |
| | | | GB | 2389759 | A | 17-12-2003 |
| | | | US | 2004022393 | A1 | 05-02-2004 |
| KR 20220132108 | A | 30-09-2022 | NONE | | | |
| CN 206797260 | U | 26-12-2017 | CN | 107139933 | A | 08-09-2017 |
| | | | CN | 107226123 | A | 03-10-2017 |
| | | | CN | 107234968 | A | 10-10-2017 |
| | | | CN | 206797260 | U | 26-12-2017 |
| | | | CN | 207029159 | U | 23-02-2018 |
| | | | CN | 207029160 | U | 23-02-2018 |
| | | | CN | 207979669 | U | 19-10-2018 |
| EP 3505051 | A1 | 03-07-2019 | NONE | | | |

EPO FORM P0459

For more details about this annex : see Official Journal of the European Patent Office, No. 12/82